# EUROPEAN PATENT SPECIFICATION

(11) **EP 0 800 371 B1**
(45) Date of publication and mention of the grant of the patent: **04.04.2001**
(21) Application number: 95944184.1
(22) Date of filing: 21.12.1995
(51) Int. Cl.: A61F 13/15

(54) **ABSORBENT ARTICLE HAVING AN INTEGRAL BARRIER**
ABSORBIERENDER ARTIKEL MIT EINER INTEGRIERTEN FLÜSSIGKEITSSPERRE
ARTICLE ABSORBANT POURVU D'UNE BARRIERE SOLIDAIRE

(30) Priority: 30.12.1994 US 366868
(43) Date of publication of application: 15.10.1997
(73) Proprietor: KIMBERLY-CLARK WORLDWIDE, INC., Neenah, Wisconsin 54956 (US)
(72) Inventor: JORGENSON, Thomas, Patrick, Neenah, WI 54956 (US); SCHUTKOSKE, Lori, Sue, Butte des Morts, WI 54927 (US)
(74) Representative: Diehl, Hermann, Dr. Dipl.-Phys.
(86) International application number: US9516688
(87) International publication number: WO9620678

(56) References cited:
- EP-A- 0 130 848
- EP-A- 0 520 884
- WO-A-92/07536
- GB-A- 2 209 672
- GB-A- 2 263 914

## Description

This invention relates generally to absorbent articles for absorbing body fluid and in particular to sanitary napkins for absorbing menstrual fluid.

All manner and variety of absorbent devices or appliances have been configured for the absorption of body fluids, such as menses, and are well known. Such devices are expected to absorb the body fluid, retain the fluid within the absorbent and to prevent the discharged body fluids from soiling the person's body and/or adjacent clothing.

In the formation of such disposable absorbent devices they commonly include a liquid-permeable bodyfacing cover, an absorbent core and a liquid-impermeable backing sheet or baffle. These absorbent devices, whether utilized as diapers, incontinence garments or sanitary napkins are subject to failure. Leakage from absorbent devices is generally attributed to a high concentration of fluid absorption at the point of fluid insult. This could be the result of a sudden release of body fluid onto absorbent device, overloading its absorption capability; or the result of a prolonged, steady discharge which may have caused the absorbent material in the device to become super-saturated and unable to accept, to a large degree, additional fluids from the body. Using a sanitary napkin as an example, menses will generally migrate radially from the point of insult and will leak from the sides. This usually results in soiling the wearer's body, typically around the thigh region, and the undergarment.

EP-A-0 520 884 discloses an absorbent article comprising an absorbent positioned in an outer cover, wherein the outer cover has a thickened portion at the side edge of the article.

EP-A-0 130 848 discloses a sanitary napkin comprising an absorbent pad and flaps extending from the longitudinal edges of the absorbent pad. The flaps have a flexible axis about which they fold on itself when the sanitary napkin is in use.

WO-A-92 07 536 shows an absorbent article comprising an absorbent pad, placed in a casing, flexible side flaps and uplifted liquid barriers on that side of the article which faces the wearer when in use.

However, in the area of sanitary napkins, it has been suggested that at least 20-25 percent of all sanitary napkins experience side leakage. One reason for this is that, when worn, the sanitary napkin can become deformed due to dynamic forces generated as the wearer moves or alters her stationary position. The greatest deformation normally occurs within that part of the article which, in use, is located in the narrowest space between the wearer's thighs. Generally, the sanitary napkin deforms by bunching, twisting, and roping which are all well known in the art. As a result of the deformation the surface area of the sanitary napkin is greatly reduced.

To overcome the problem of side leakage, sanitary napkins have been constructed having elasticized sides that urge the sides upward or cause the sanitary napkin to form a cup shape.

Another method of preventing side leakage has been to extend wings, flaps or panels (hereinafter wings) from the edges of the sanitary napkin. the wings generally extend over the edges of the undergarment and are intended to adhere to the underside of the crotch portion or to themselves. The wings typically assist the garment adhesive, if present, to hold the sanitary napkin in position during use. To some extent, the wings counteract deformation of the sanitary napkin since they are anchored to the longitudinal edges of the undergarment. The wings also form a guard against side leakage and minimize soiling the wearer's undergarment.

In some cases, such as when the sanitary napkin is positioned crookedly, or when it becomes wrinkled or folded, wings can fold inward, partially occluding the fluid receiving surface. This diminishes the efficacy of the sanitary napkin to absorb discharged body fluids. At other times, the wings can contribute to an incidence of failure. This can occur because the wings generally are intended to form liquid barriers and seldom contain any absorbent material. Thus, the wings act as a transporting means to spread over a large area any body liquids reaching them.

Improving the performance of sanitary napkins continues to be a formidable undertaking, although a number of improvements have been made in both materials and construction. However, eliminating leakage, particularly along the inside of the thighs without compromising comfort and fit has not yet met the desired needs of the consumer.

Therefore, there remains a need for a sanitary napkin that will be comfortable to wear while decreasing the chance of side leakage associated with the use of sanitary napkins during the menstrual period.

It is the object of the present invention to overcome the above described problems and to provide an absorbent article which meets the above described needs.

This object is solved by the absorbent article according to independent claims 1 and 11. Further advantageous features, aspects and details of the invention are evident from the dependent claims, the description and the drawings. The claims are intended to be understood as a first, non-limiting approach to defining the invention in general terms.

Briefly, this invention relates to disposable absorbent articles, and more particularly, to sanitary napkins which are designed to absorb body fluids, such as menstrual fluid, and other excrements discharged by the body during a menstrual period. The present invention provides an absorbent article having improved side leakage protection. More particularly, the present invention provides a sanitary napkin having an integral fluid barrier positioned along the longitudinal sides thereof.

Broadly, this invention provides an absorbent structure having an integral barrier device. According to a first aspect, the present invention provides an absorbent article having a bodyfacing surface, a liquid impermeable baffle or surface, and longitudinal side edges being gathered or wound to form integral longitudinal side barriers. The longitudinal side edges preferably extend laterally outward from a longitudinal central axis of the absorbent structure. The absorbent article or structure may have a garment-facing surface (106) to which said liquid impermeable baffle is secured, wherein one of said absorbent or said baffle may have a greater width and extends laterally outwardly to define said longitudinal side edges.

According to a second aspect, the present invention provides an absorbent article comprising a liquid-permeable cover having a bodyfacing surface; a liquid-impermeable baffle having a garment-facing surface; and an absorbent positioned between said cover and said baffle, said absorbent preferably having longitudinal side portions wherein at least one of said cover or said baffle extend laterally beyond said longitudinal side portions to form longitudinal side edges which are spirally wound inward to form integral longitudinal side barriers. In one embodiment, an absorbent structure or article is provided having a longitudinal central axis, a liquid-permeable bodyfacing surface superposed over a liquid-impermeable garment-facing surface. The bodyfacing surface and the garment-facing surface extend laterally outward relative to the longitudinal axis to form longitudinal edges. The longitudinal edges are wound to form integral, longitudinal barriers.

In another embodiment, an absorbent article is provided having an absorbent adapted to reside adjacent to the wearer. The absorbent has a liquid-permeable bodyfacing surface and a garment-facing surface. Secured to the garment-facing surface is a liquid-impermeable baffle. Preferably, the baffle has a width greater than the absorbent and extends laterally outward to define longitudinal edges of the absorbent article. The longitudinal edges are gathered to form integral, longitudinal barriers. The absorbent article can include other features such as a cover and a resilient layer.

In another embodiment, a sanitary napkin is provided having a liquid-permeable cover with a bodyfacing surface, a liquid-impermeable baffle and an absorbent positioned between the cover and the baffle. The absorbent has longitudinal side portions. The cover and/or baffle extend laterally outward from the longitudinal side portions to define a pair of longitudinal edges of the sanitary napkin. The longitudinal edges are gathered and preferably, spirally wound to form integral longitudinal barriers. The integral barriers include tensioning means for imparting an arcuate shape to the absorbent structure. By "arcuate" it is meant that when the absorbent structure is placed on a flat or planar surface at least one of the transverse ends will be spaced above the surface.

According to a general aspect, this invention provides an absorbent structure having improved side leakage protection and being comfortable to wear. More specifically, this invention provides an improved absorbent article having integral fluid barriers.

According to another aspect, this invention provides an absorbent article having integral longitudinal barriers formed by the gathering of the laterally extending, longitudinal sides of the absorbent article.

According to a further aspect, this invention provides a curved sanitary napkin having an integral barrier. The barrier is formed by rolling the longitudinal sides of the sanitary napkin. The integral barrier has secured to it a tensioning means for bending the integral barrier into a concave shape toward the bodyfacing surface of the sanitary napkin.

Fig. 1 is a top perspective view of an absorbent structure showing longitudinal side portions extending laterally.

Fig. 2 is a top perspective view of the bodyfacing surface of the absorbent structure shown Fig. 1 illustrating the resulting configuration of the side barriers.

Fig. 3 is an enlarged cross-sectional view of the absorbent structure shown in Fig. 2 taken along line 3--3 of Fig. 2.

Fig. 4 is a top perspective view of an absorbent article illustrating non-linear longitudinal side portions extending laterally.

Fig. 5 is a cross-sectional view of the absorbent article shown in Fig. 4 illustrating the longitudinally extending sides prior to being gathered to form the side barriers.

Fig. 6 is an enlarged cross-sectional view of the absorbent article shown in Fig. 4 illustrating the resulting configuration of the side barrier.

Fig. 7 is a top plan view of the bodyfacing surface of another absorbent article prior to forming the integral side barrier, illustrating non-linear longitudinal side portions extending laterally outward from an absorbent and tensioning means for bending the absorbent structure.

Fig. 8 is a top perspective view of the absorbent structure shown in Fig. 7 illustrating the resulting concave configuration of the absorbent structure.

Fig. 9 is an enlarged cross-sectional view of the absorbent article taken along line 9--9 of Fig. 9.

Although described hereafter as a sanitary napkin, it is understood that the invention can be adapted for use in disposable diapers, adult incontinence devices, training pants and the like. In the various views, similar components therein will be differentiated by the use of a prime (') symbol.

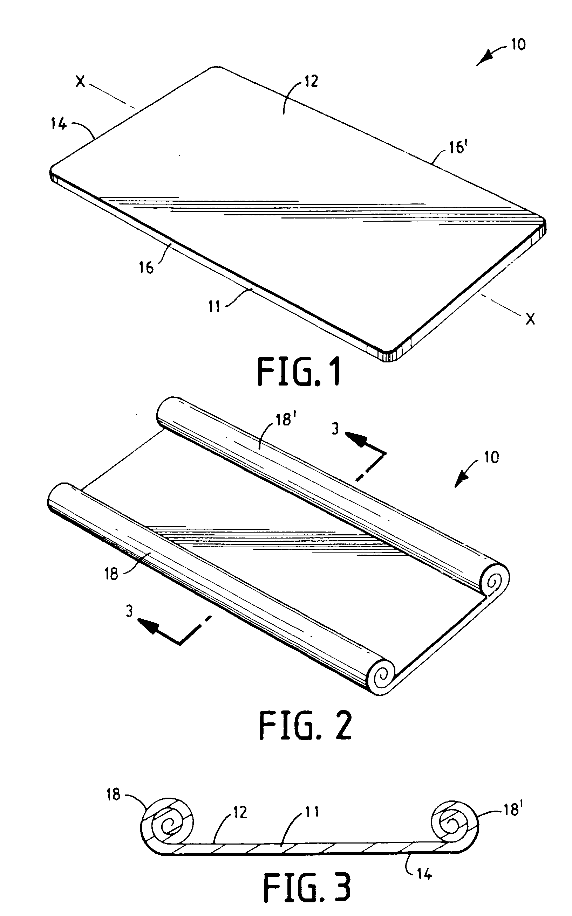

Referring to Fig. 1-3, an absorbent structure 10 is provided having a longitudinal central axis X--X, an absorbent 11, a liquid-permeable bodyfacing surface 12 and a liquid-impermeable garment-facing surface 14. The bodyfacing surface 12 is superposed over at least a portion of the garment-facing surface 14. Preferably, the bodyfacing surface 12 and the garment-facing 14 are coextensive.

Extending laterally outward from the longitudinal central axis X--X of the absorbent structure 10 are longitudinal side edges 16 and 16'. The longitudinal side edges 16 and 16' are gathered together to form integral longitudinal side barriers 18 and 18'. Preferably, the side barriers 18 and 18' are formed by rolling the longitudinal edges 16 and 16', and more preferably, spirally winding the longitudinal side edges 16 and 16' inward toward the longitudinal central axis X--X. The longitudinal edges 16 and 16' can be spirally wound toward the garment-facing surface 14 but preferably are wound toward the bodyfacing surface 12.

Spirally winding the longitudinal side edges 16 and 16' is advantageous because it produces an outer configuration that is rounded and less likely to chaff and irritate the sensitive tissues in the thigh and crotch region of the wearer during use. Spirally winding the longitudinal edges 16 and 16' also produces a barrier 18 or 18' that is uniform. Other methods suitable for forming a barrier include folding the longitudinal edges 16 and 16' a predetermined number of times along one or more longitudinal fold lines to produce an accordion configuration (not shown).

The absorbent structure 10 can be produced by using a known absorbent, discussed in greater detail below. The garment-facing surface 14 is then rendered hydrophobic by coating or impregnating the absorbent with a suitable material. One such material is a liquid latex that, when dried, forms a hydrophobic surface.

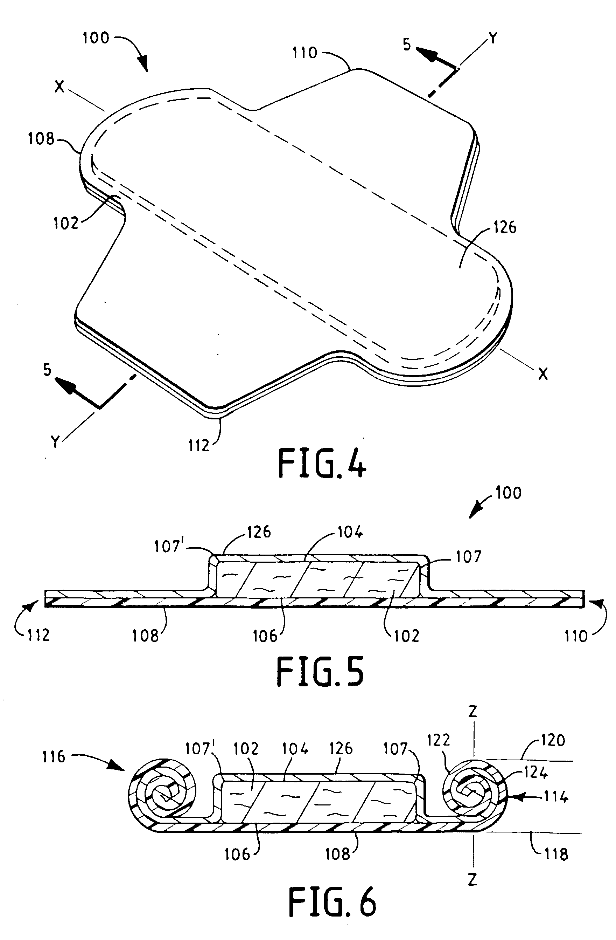

Referring to Figs. 4-9, disposable absorbent articles 100 and 200 of the present invention are illustrated in the form of a sanitary napkin 100 and 200 and are meant to be exemplary only and should not be deemed as limiting the scope of the present invention. Typically, a sanitary napkin is wom by a female to absorb body fluids, such as menses, blood, urine and other body excrements discharged during a menstrual period.

Referring to Figs. 4-6, a sanitary napkin 100 is provided having an absorbent 102. The absorbent 102 has a bodyfacing surface 104, a garment-facing surface 106 and longitudinal side portions 107 and 107'.

The bodyfacing surface 104 is disposed toward the wearer's body and adapted to reside adjacent to the wearer in use. The garment-facing surface 106 is disposed distal from the bodyfacing surface 104 and is adapted to reside adjacent to the wearer's undergarment. Secured to a garment-facing surface 106 is a liquid-impermeable baffle 108. Desirably, the baffle 108 has a width greater than the absorbent 102, extending laterally beyond the longitudinal side portions 107 and 107' to define longitudinal side edges 110 and 112. The longitudinal edges 110 and 112 can have any length ranging from a few millimeters to the entire length of the sanitary napkin 100. As used herein, the term "length" means the distance measured from one transverse end to the other transverse end and parallel to the longitudinal axis X--X of the sanitary napkin 100. The longitudinal side edges 110 and 112 can be asymmetrical about a transverse axis Y--Y but preferably they are symmetrical. The longitudinal side edges 110 and 112 can be linear, nonlinear or have some other geometric configurations such as triangular, semi-circular, etc.

Each longitudinal side edge 110 and 112 is gathered to form an integral longitudinal side barrier 114 and 116. Since the barriers 114 and 116 are similar in construction, only side barrier 114 will be discussed in detail, but it is to be understood as applying equally to side barrier 116. The side barrier 114 can be formed by folding the baffle 108 one or more times along a longitudinal line to form an accordion configuration barrier (not shown). Preferably, the side barrier 114 is formed by rolling the longitudinal side edge 110, and more preferably, spirally winding the longitudinal side edge 110 inward toward the longitudinal central axis X--X, and most preferably the longitudinal side edge 110 is spirally wound toward the bodyfacing surface 104. The length of the side barrier 114 is substantially determined by the length of the longitudinal side edge 110. The height of the integral side barrier 114 can be adapted for its intended use. The height of the barriers 114 is measured from a lower most surface 118 to an upper most surface 120. Since one intended function of the side barrier 114 is to improve side leakage protection, the side barrier 114 should have a height where the upper most surface 120 extends above the plane of the garment-facing surface 106 and preferably, it is substantially parallel to the plane of the bodyfacing surface 104, and more preferably, it is above the plane of the bodyfacing surface 104.

Referring to Fig. 6, the side barrier 114 has a vertical central axis Z-Z that substantially divides the side barrier 114 into an inside segment 122 and an outside segment 124. The inside segment 122 is positioned proximate the longitudinal side portion 107 while the outside segment 124 is positioned distally from the longitudinal side portion 107. The side barrier 114 can be hydrophilic, hydrophobic or a combination thereof. Preferably, the inside segment 122 is hydrophilic and the outside segment 124 is hydrophobic. This arrangement allows the side barrier 114 to enhance the absorbent capacity of the sanitary napkin 100 without adding substantial absorbent material to the absorbent 102. Another advantage to this arrangement is that placement of the sanitary napkin 100 in the crotch portion of the undergarment does not have to be as precise to maintain the efficacy of the sanitary napkin 100. The inside segment 122 can be positioned so that it resides adjacent to the longitudinal side portion 107 or spaced a distance therefrom up to about 25 millimeters, and preferably is spaced a distance of about 2 millimeters to about 15 millimeters from the longitudinal side portion 107.

Referring again to Fig. 4, one skilled in the absorbent art will recognize that by utilizing this invention one can construct an integral side barrier 114 having unique configurations. Depending upon the lateral extension of the longitudinal side edge 110, for example, by using an irregularly shaped longitudinal side edge 110, the side barrier 114 can have varied height dimensions. In this manner, one can customize the location of the barrier, improve side leakage protection in those areas where needed, and enhance the comfort of the sanitary napkin 100.

In the sanitary napkin 100, the absorbent 102 provides a means for absorbing the menstrual fluid. The total absorbent capacity of the absorbent 102 should be compatible with the predetermined exudate loading in the intended use of the sanitary napkin 100. Furthermore, the size and shape of the absorbent 102 can be varied. For example, the absorbent 102 can be rectangular, oval, racetrack or any other geometrical shape known in the absorbent art.

The absorbent 102 is generally made from one or more materials that, in combination, are substantially hydrophilic, compressible, conformable and non-irritating to the wearer's skin. Acceptable materials are well known in the art and include, for example, various natural or synthetic fibers, wood pulp fibers, regenerated cellulose or cotton fibers, or a blend of pulp and other fibers, meltblown polymer, such as polyester, and polypropylene. The absorbent layers may also be comprised of other well-known materials used in absorbent articles, including multiple layers of cellulose wadding, rayon fibers, cellulose sponge, hydrophilic synthetic sponge, such as polyurethane, and the like.

The absorbent 102 may contain superabsorbents which are effective in retaining body fluids. Superabsorbents have the ability to absorb a large amount of fluid in relation to their own weight. Typical superabsorbents used in absorbent articles, such as sanitary napkins, can absorb anywhere from about 5 to about 60 times their weight in body fluids.

Referring again to Fig. 6, the baffle 108 may be constructed from any desired material that is liquid-impermeable on the undergarment facing side and preferably will permit the passage of air and moisture vapor out of the sanitary napkin 100 while blocking the passage of body fluids. A good material is a micro-embossed, polymeric film, such as polyethylene or polypropylene having a thickness of about 0.025 to 0.13 millimeters (0.001 to about 0.005 of an inch). Bi-component films can also be used as well as woven and nonwoven fabrics which have been treated to render them liquid-impermeable. Another suitable material is a closed cell polyolefin foam. For example, a closed cell polyethylene foam having a thickness ranging from about 0.5 millimeters to about 10 millimeters.

The sanitary napkin 100 may include other features such as cover 126. Generally, the cover 126 is provided for comfort and conformability and directs fluid to the underlying absorbent 102. The cover 126 can be constructed from a relatively non-absorbing fluid pervious material. The cover 126 can be constructed of any woven or nonwoven material which is easily penetrated by body fluid contacting its surface. Preferably, the cover 126 is made of a material which allows the passage of fluid without wicking it appreciably in a horizontal plane parallel to the cover 126. Furthermore, the cover 126 should retain little or no fluid in its structure so that it provides a relatively dry surface next to the skin. Generally, the cover 126 is a single sheet of material having a width sufficient to overlie the bodyfacing surface 104 of the absorbent 102. Preferably, the cover 126 extends to the longitudinal side edges 110 and 112 and is secured to the baffle 108. The cover 126 can be secured to the baffle 108 using any suitable method that does not leave a hard, uncomfortable residue that would be annoying to the wearer. Methods for joining the various materials are well known to those skilled in the art and include the use of pressure sensitive adhesives, hot melt adhesives, double-sided tape, ultra sonic bonding, and heat sealing to name a few. Adhesives, such as the hot melt adhesives, can be applied in a uniform manner and as a continuous or noncontinuous layer.

The cover 126 can be constructed of bonded carded webs of polyester, polypropylene, polyethylene, nylon, or other heat-bondable fibers. Other polyolefins, such as copolymers of polypropylene and polyethylene, liner low-density polyethylene, finely perforated film webs and net material also work well. Other suitable materials are composite materials of polymer and a nonwoven fabric material. The composite sheets are generally formed by extrusion of polymer onto a web of spunbond material to form an integral sheet. This material is preferred because the outer fabric surface is not irritating to the skin of the wearer and has a cushion feel.

Another preferred material for the cover 126 is a spunbond web of polypropylene. The web can contain about 1 to 6 percent titanium dioxide pigment to give it a clean white appearance. The most preferred polypropylene webs have a weight of between about 10 and 40 grams per square meter. Desirably, the weight is between about 20 and about 35 grams per square meter.

The liquid-permeable cover 126 can also contain a plurality of apertures (not shown) formed therein. Such apertures should be sized so fluid can pass through the cover 126 and into the absorbent 102. The apertures can be arranged longitudinally or can be zoned or localized to the area intended to be insulted with the body fluid, if desired. The apertures are intended to increase the rate at which body fluids can penetrate down into the absorbent 102. This helps maintain a perceivably drier surface for the cover 126 than when the apertures are not present. Therefore, while the apertures are not essential, a functional advantage is obtained by their use.

The liquid-permeable cover 126 can also be treated with a surfactant to make it more hydrophilic and, thereby, aid in the absorption of the liquid. The surfactant can include topical additions or internally applied materials like polysiloxanes.

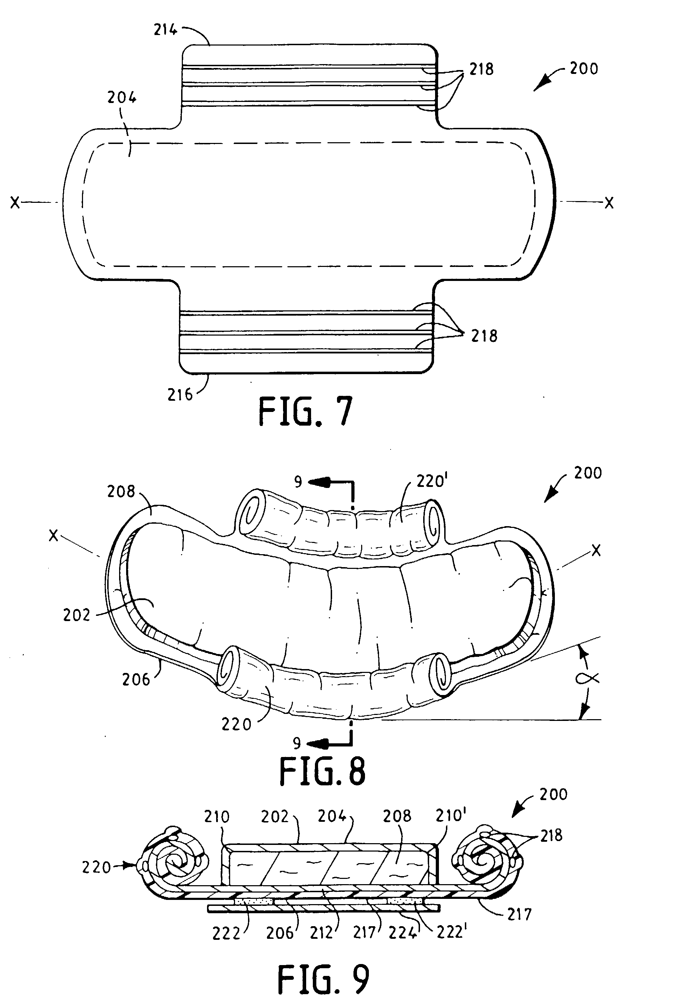

Referring to Figs. 7-9 a sanitary napkin 200 is depicted illustrating an alternative embodiment of this invention. The sanitary napkin 200 has a liquid-permeable cover 202 with a bodyfacing surface 204, a liquid-impermeable baffle 206 and an absorbent 208. The absorbent 208 has two longitudinal side portions 210 and 210'.

Referring to Fig. 9, the sanitary napkin 200 further includes a resilient layer 212. The resilient layer is positioned between the cover 202 and baffle 206, preferably, it is the adjacent to the absorbent 208, and more preferably it is between the baffle 206 and the absorbent 208 and extends laterally outward from the longitudinal side portions 210 and 210'. At least one of the cover 202, baffle 206 and/or resilient layer 212 is slightly more expansive than the absorbent 208 and extend laterally outward from the longitudinal side portion 210 to form longitudinal side edges 214 and 216. The resilient layer 212 can be hydrophilic or hydrophobic and can provide added absorbency and/or resiliency to the sanitary napkin 200.

The resilient layer 212 can extend beyond the longitudinal side portions 210 and 210' and may be coterminous with the cover 202 or the baffle 206. The resilient layer 212 may comprise a thin layer of absorbent material, such as, tissue, fabric or the like, made of cellulosic fibers. Because such material is provided as a safety measure and is only required to contain escaped fluid, it does not need to have a significant absorbent capacity relative to the absorbent 208. Another material is a meltblown polypropylene layer having a thickness of about 0.3 mm to about 1.0 mm, preferably about 0.4 to about 0.6 and having a weight of about 30 grams per square meter (gsm) to about 200 gsm, and preferably about 50 gsm to about 100 gsm. Such material is available from the Kimberly-Clark Corporation having offices at 401 North Lake Street, Neenah, Wisconsin and having a raw material specification number of 6103. A preferred material is a multicomponent, nonwoven polymer fabric as described in the patent application having U.S. Serial No. 07/933,444 filed on August 21, 1992 entitled "Nonwoven Multicomponent Polymer Fabric and Method of Making Same" and assigned to the present assignee, the entire disclosure of which is incorporated herein by reference.

A suitable hydrophobic material for use as a resilient layer 212 is any light weight polyolefin foam material. Such foams can be open or closed cell. Suitable foams include polyurethane and crosslinked or non-crosslinked polyolefin foams. Desirably, the foam is a polypropylene or a polyethylene foam, with polyethylene being preferred. Particularly preferred is a non-crosslinked polyethylene foam.

The foam can have a thickness ranging from about 0.381 millimeters to about 6.35 millimeters, preferably from about 0.51 millimeters to about 1.54 millimeters, and more preferably, from about 0.76 millimeters to about 1.27 millimeters, and most preferably, 0.76 millimeters to about 1.02 millimeters.

The foam can have a density ranging from about 0.0225 gm/cm³ to about 0.0962 gm/cm³, preferably ranging from about 0.0322 gm/cm³ to about 0.0642 gm/cm³, and most preferably from about 0.0354 gm/cm³ to about 0.0482 gm/cm³.

Since the longitudinal side edge 214 and 216 are similar only side edge 214 will be described further. The longitudinal side edge 214 can have any length ranging from a few millimeters to extending the entire length of the sanitary napkin 200. The longitudinal side edge 214 can be linear, non-linear or have any other geometric configuration such as semicircular, triangular and the like.

Disposed on the laterally-extending material between the longitudinal side portion 210 and the longitudinal side edge 214 is at least one longitudinal oriented tensioning member 218. Tensioning member 218 can be secured to either the bodyfacing surface 204 of the cover 202 or to a garment-facing surface 217 of the baffle 206. Alternatively, the tensioning member 218 can be secured between the cover 202 and baffle 206. The tensioning member 218 can be secured to the laterally-extending material by means well known in the art, such as, for example, using adhesives or a discontinuous ultrasonic bond.

The tension member 218 can be one or more elastic strips. If more than one elastic strip is utilized, it is preferred that the strips be spaced apart. The elastic strip is under tension prior to its securement to the laterally-extending material. Relative to its relaxed state, the elastic strip can be elongated up to 50% and preferably 100% prior to securing the elastic strip the to laterally-extending material. For the purposes of comfort, it is desired that the elastic strip have a width ranging from about 1 mm to about 7 mm in width, and preferably about 4 mm. Elastic having a width much greater than about 7 mm tends to chafe and become uncomfortable for the wearer.

Another method of introducing elastic is by utilizing an extrudable elastic which can be initially extruded as a liquid and upon cooling becomes both an adhesive and an elastic. This eliminates the separate step of adhesively bonding the strips of elastic to either side. An example of such a product is described in U.S. Patent No. 4,259,220 assigned to H.D. Fuller Co. in St. Paul, Minnesota, the disclosure of which is incorporated herein by reference and made a part hereof.

The longitudinal side edge 214 is then gathered inward toward the absorbent 208 and more preferably, toward the bodyfacing surface 204 to form a liquid side barrier 220 . Preferably, the side barrier 220 is formed by rolling and more preferably, spirally winding the longitudinal side edge 214. To assist in rolling the longitudinal side edge 214, a formed member can be used, (not shown). The formed member can have any geometrical configuration but preferably, is curved to facilitate rolling. The formed member can have a width dimension of from about 2 mm to about 12 mm. A preferred formed member is a rod shaped polyurethane foam having a diameter of about 3 mm to about 5 mm and a length substantially coinciding with the length of the longitudinal side edge 214.

The tensioning member 218 advantageously imparts to the sanitary napkin 200 a concave shape to the bodyfacing surface 204. This preferred configuration will provide a better fit to the body of the wearer. Preferably, the tension member 218 urges the side barrier 220 to rise forming an arcuate surface. As used herein the term "arcuate" means that when one transverse end of the sanitary napkin 200 is placed on a planar surface, the angle formed by the outer profile of other transverse end and the plane upon which the sanitary napkin 200 rests is between 10 degrees and 90 degrees.

The sanitary napkin 10 is about 150 millimeters (mm) to about 300 mm long and about 50 mm to about 175 mm wide at its widest point. The sanitary napkin 10 has an hourglass configuration but can include such shapes as rectangular, oval, racetrack, dogbone and the like.

Referring again to Fig. 9, the sanitary napkin 200 can be provided with attachment adhesive 222 and 222' applied to the garment-facing surface 217 of the baffle 206. The adhesive 222 and 222' can be made from any known pressure-sensitive material. As used herein the term "pressure sensitive" refers to any releasable adhesive or releasable tenacious means. Adhesive compositions suitable for sanitary napkins, includes, for example, the water-based pressure-sensitive adhesives such as acrylate adhesives. Alternatively, the adhesive may comprise rapid setting thermoplastic "hot melt", rubber adhesives or two-sided adhesive tape. As is customary in the art, the adhesive 222 and 222' can be covered by a protective release liner 224 such as a Kraft paper that is silicone coated.

In use, the wearer removes the release liner 224 and attaches the sanitary napkin 200 to the inside surface of her undergarment. The adhesive strips 222 and 222' allow the sanitary napkin 200 to remain in position to receive discharged liquids.

## Claims

1. An absorbent article (10; 100; 200) comprising:
an absorbent (11; 102; 208) having a bodyfacing surface (12; 104);
a liquid-impermeable baffle (14; 108; 206); and
longitudinal side edges (16, 16'; 110, 112; 214, 216)
characterized in that
said longitudinal side edges (16, 16'; 110, 112; 214, 216) are spirally wound inward to form integral longitudinal side barriers (18, 18'; 114, 116; 220, 220').

2. The absorbent article of claim 1, wherein said liquid impermeable baffle (14) forms a surface of the absorbent article, and
wherein said longitudinal side edges (16, 16') extend laterally outward from a longitudinal central axis of the absorbent structure.

3. The absorbent article of one of the preceding claims, said absorbent (102; 208) having a garment-facing surface (106), said liquid-impermeable baffle (108; 206) being secured to said garment-facing surface (106), one of said absorbent or said baffle having a greater width and extending laterally outwardly to define said longitudinal side edges (110, 112; 214, 216).

4. The absorbent article of one of the preceding claims wherein said absorbent (102) is wider than said baffle (108).

5. The absorbent article of one of claims 1 to 3 wherein said baffle (108) is wider than said absorbent (102).

6. The absorbent article of one of the preceding claims wherein said longitudinal side edges (110, 112) are spirally wound inward toward said absorbent (102) to form said integral barriers (114, 116).

7. The absorbent article of one of the preceding claims further comprising a liquid-permeable cover (126) secured to said bodyfacing surface (104) of said absorbent (102).

8. The absorbent article of one of the preceding claims wherein said integral barriers (114, 116) have an upper most portion extending above the plane of said baffle (108).

9. The absorbent article of one of the preceding claims further comprising tensioning means (218) for imparting a concave curvature along a longitudinal axis toward said bodyfacing surface (204), said tensioning means (218) being secured to at least one of said integral barriers (220, 220').

10. The absorbent article of one of the preceding claims further comprising a resilient member positioned between said bodyfacing surface (204) and said baffle (206) wherein at least one of said first member, said baffle (206) or said resilient layer (212) has a greater width and extends laterally outwardly to define said longitudinal side edges (214, 216), said edges being spirally wound inward to form said integral longitudinal side barriers (220, 220').

11. The absorbent article of claim 1, further comprising a liquid-permeable cover (126; 202) having a bodyfacing surface (204), wherein said liquid-impermeable baffle has a garment-facing surface (106), and wherein said absorbent (102; 208) is positioned between said cover (126; 202) and said baffle (108; 206), said absorbent having longitudinal side portions (107; 210) wherein at least one of said cover (126; 202) or said baffle (108; 206) extend laterally beyond said longitudinal side portions (107; 210) to form said longitudinal side edges (110, 112; 214, 216).

12. The absorbent article of one of the preceding claims wherein said longitudinal side edges (110, 112) are spirally wound inward toward said baffle (108).

13. The absorbent article of one of the preceding claims wherein said longitudinal side edges (110, 112) are spirally wound toward said bodyfacing surface (104) to form said barrier.

14. The absorbent article of one of claims 7 to 13 wherein both said cover (126) and said baffle (108) extend laterally beyond said longitudinal side portions (107) to form said longitudinal side edges (110, 112).

15. The absorbent article of one of claims 7 to 14 wherein said integral barriers (114, 116) have an upper most portion that extends above the plane of said bodyfacing surface of said cover (126).

16. The absorbent article of one of claims 7 to 15 further comprising a resilient layer (212) positioned between said cover (202) and said baffle (206).

17. The absorbent article of claim 16 wherein said resilient layer (212) is hydrophilic.

18. The absorbent article of one of claims 16 or 17 wherein said resilient layer (212) extends laterally outward beyond said longitudinal side portions (210) of said absorbent (208).

19. The absorbent article of one of claims 11 to 18 wherein said integral side barrier (114) has a vertical center line dividing said integral side barrier into an inside segment (122) and an outside segment (124), said inside segment (122) is positioned proximate said longitudinal side portion (107) and said outside segment (124) is positioned distally from said longitudinal side portion (107), said inside segment (122) being at least partially hydrophilic.

20. The absorbent article of claim 19 wherein said inside segment (122) is positioned adjacent to said longitudinal side portion (107).

21. The absorbent article of claim 19 or 20 wherein said inside segment (122) is spaced a predetermined distance from said longitudinal side portion (107).

22. The absorbent article of one of claims 11 to 21 wherein said longitudinal side portions (107) are non-linear.

23. The absorbent article of one of the preceding claims further comprising tensioning means (218) for imparting a concave curvature along a longitudinal axis toward said bodyfacing surface (204), said tensioning means (218) being secured to at least one of said integral barriers (220, 220').

24. The absorbent article of one of the preceding claims, being a sanitary napkin.

25. The absorbent article of one of claims 16 to 24, wherein said absorbent (208) is positioned adjacent to said resilient layer (212), said absorbent having a longitudinal side portion (210) and at least one of said cover (202), said baffle (206) or said resilient layer (212) extend laterally beyond said longitudinal side portion (210) to form the longitudinal side edge (216), said longitudinal side edge (216) being spirally wound inward to form an integral longitudinal side barrier (220).

26. The absorbent article of one of the preceding claims wherein said longitudinal side edge (216) is non-linear.

27. The absorbent article of one of claims 25 or 26 wherein said laterally extending sides are spirally wound inward toward said bodyfacing surface (104; 204) and said integral side barrier (114, 116; 220, 220') extends above the plane of said baffle (108; 206).

## Patentansprüche

1. Saugfähiger Artikel (10; 100; 200), umfassend:
einen Saugkörper (11; 102, 208) mit einer körperseitigen Oberfläche (12; 104);
eine flüssigkeitsundurchlässige Sperrschicht (14; 108; 206); und
Längsseitenkanten (16, 16', 110, 112; 214; 216);
dadurch gekennzeichnet, dass
die Längsseitenkanten (16, 16', 110, 112; 214; 216) zur Bildung integraler Längsseitenbarrieren (18, 18'; 114, 116, 220, 220') spiralförmig nach innen gerollt sind.

2. Saugfähiger Artikel nach Anspruch 1, wobei die flüssigkeitsundurchlässige Sperrschicht (14) eine Oberfläche des saugfähigen Artikels bildet, und wobei die Längsseitenkanten (16, 16') sich von einer Längsmittelachse der saugfähigen Struktur seitlich nach außen erstrecken.

3. Saugfähiger Artikel nach einem der vorangehenden Ansprüche, wobei der Saugkörper (102; 208) eine bekleidungsseitige Oberfläche (106) aufweist, wobei die flüssigkeitsundurchlässige Sperrschicht (108; 206) an der bekleidungsseitigen Oberfläche (106) befestigt ist, wobei entweder der Saugkörper oder die Sperrschicht eine größere Breite aufweist und sich seitlich nach außen erstreckt, um die Längsseitenkanten (110, 112; 214, 216) zu definieren.

4. Saugfähiger Artikel nach einem der vorangehenden Ansprüche, wobei der Saugkörper (102) breiter als die Sperrschicht (108) ist.

5. Saugfähiger Artikel nach einem der Ansprüche 1 bis 3, wobei die Sperrschicht (108) breiter als der Saugkörper (102) ist.

6. Saugfähiger Artikel nach einem der vorangehenden Ansprüche, wobei die Längsseitenkanten (110, 112) spiralförmig nach innen zu dem Saugkörper (102) zur Bildung der integralen Barrieren (114, 116) gerollt sind.

7. Saugfähiger Artikel nach einem der vorangehenden Ansprüche, umfassend eine flüssigkeitsdurchlässige Abdeckung (126), die an der körperseitigen Oberfläche (104) des Saugkörpers (102) befestigt ist.

8. Saugfähiger Artikel nach einem der vorangehenden Ansprüche, wobei die integralen Barrieren (114, 116) einen obersten Abschnitt aufweisen, der sich über die Ebene der Sperrschicht (108) hinaus erstreckt.

9. Saugfähiger Artikel nach einem der vorangehenden Ansprüche, des Weiteren umfassend Spannmittel (218), um eine konkave Krümmung entlang einer Längsachse zu der körperseitigen Oberfläche (204) zu bilden, wobei das Spannmittel (218) an mindestens einer der integralen Barrieren (220, 220') befestigt ist.

10. Saugfähiger Artikel nach einem der vorangehenden Ansprüche, des Weiteren umfassend ein elastisches Element, das zwischen der körperseitigen Oberfläche (204) und der Sperrschicht (206) angeordnet ist, wobei zumindest eines von dem ersten Element, der Sperrschicht (206) oder der elastischen Schicht (212) eine größere Breite aufweist und sich seitlich nach außen erstreckt, um die Längsseitenkanten (214, 216) zu definieren, wobei die Kanten zur Bildung der integralen Längsseitenbarrieren (220, 220') spiralförmig nach innen gerollt sind.

11. Saugfähiger Artikel nach Anspruch 1, des Weiteren umfassend eine flüssigkeitsdurchlässige Abdeckung (126; 202) mit einer körperseitigen Oberfläche (204), wobei die flüssigkeitsundurchlässige Sperrschicht eine bekleidungsseitige Oberfläche (106) aufweist, und wobei der Saugkörper (102; 208) zwischen der Abdeckung (126; 202) und der Sperrschicht (108; 206) angeordnet ist, wobei der Saugkörper Längsseitenabschnitte (107; 210) aufweist, wobei sich mindestens eine von der Abdeckung (126; 202) oder der Sperrschicht (108; 206) seitlich über die Längsseitenabschnitte (107; 210) zur Bildung der Längsseitenkanten (110, 112; 214, 216) hinaus erstreckt.

12. Saugfähiger Artikel nach einem der vorangehenden Ansprüche, wobei die Längsseitenkanten (110, 112) spiralförmig nach innen zu der Sperrschicht (108) gerollt sind.

13. Saugfähiger Artikel nach einem der vorangehenden Ansprüche, wobei die Längsseitenkanten (110, 112) spiralförmig zu der körperseitigen Oberfläche (104) zur Bildung der Barriere gerollt sind.

14. Saugfähiger Artikel nach einem der Ansprüche 7 bis 13, wobei sowohl die Abdeckung (126) als auch die Sperrschicht (108) zur Bildung der Längsseitenkanten (110, 112) seitlich über die Längsseitenabschnitte (107) hinaus verlaufen.

15. Saugfähiger Artikel nach einem der Ansprüche 7 bis 14, wobei die integralen Barrieren (114, 116) einen obersten Abschnitt aufweisen, der sich über die Ebene der körperseitigen Oberfläche der Abdeckung (126) hinaus erstreckt.

16. Saugfähiger Artikel nach einem der Ansprüche 7 bis 15, des Weiteren umfassend eine elastische Schicht (212), die zwischen der Abdeckung (202) und der Sperrschicht (206) angeordnet ist.

17. Saugfähiger Artikel nach Anspruch 16, wobei die elastische Schicht (212) hydrophil ist.

18. Saugfähiger Artikel nach einem der Ansprüche 16 oder 17, wobei die elastische Schicht (212) sich seitlich nach außen über die Längsseitenabschnitte (210) des Saugkörpers (208) hinaus erstreckt.

19. Saugfähiger Artikel nach einem der Ansprüche 11 bis 18, wobei die integrale Seitenbarriere (114) eine vertikale Mittellinie aufweist, welche die integrale Seitenbarriere in ein inneres Segment (122) und ein äußeres Segment (124) teilt, wobei das innere Segment (122) nahe dem Längsseitenabschnitt (107) angeordnet ist, und das äußere Segment (124) distal von dem Längsseitenabschnitt (107) angeordnet ist, wobei das innere Segment (122) zumindest teilweise hydrophil ist.

20. Saugfähiger Artikel nach Anspruch 19, wobei das innere Segment (122) neben dem Längsseitenabschnitt (107) angeordnet ist.

21. Saugfähiger Artikel nach Anspruch 19 oder 20, wobei das innere Segment (122) mit einem vorbestimmten Abstand von dem Längsseitenabschnitt (107) beabstandet ist.

22. Saugfähiger Artikel nach einem der Ansprüche 11 bis 21, wobei die Längsseitenabschnitte (107) nichtlinear sind.

23. Saugfähiger Artikel nach einem der vorangehenden Ansprüche, des Weiteren umfassend Spannmittel (218), um eine konkave Krümmung entlang einer Längsachse zu der körperseitigen Oberfläche (204) zu bilden, wobei das Spannmittel (218) an mindestens einer der integralen Barrieren (220, 220') befestigt ist.

24. Saugfähiger Artikel nach einem der vorangehenden Ansprüche, der eine Damenbinde ist.

25. Saugfähiger Artikel nach einem der Ansprüche 16 bis 24, wobei der Saugkörper (208) neben der elastischen Schicht (212) angeordnet ist, wobei der Saugkörper einen Längsseitenabschnitt (210) aufweist und sich mindestens eine von der Abdeckung (202), der Sperrschicht (206) oder der elastischen Schicht (212) seitlich über den Längsseitenabschnitt (210) zur Bildung der Längsseitenkante (216) hinaus erstreckt, wobei die Längsseitenkante (216) zur Bildung einer integralen Längsseitenbarriere (220) spiralförmig nach innen gerollt ist.

26. Saugfähiger Artikel nach einem der vorangehenden Ansprüche, wobei die Längsseitenkante (216) nichtlinear ist.

27. Saugfähiger Artikel nach einem der Ansprüche 25 oder 26, wobei die sich seitlich erstreckenden Seiten spiralförmig nach innen zu der körperseitigen Oberfläche (104; 204) gerollt sind und die integrale Seitenbarriere (114, 116; 220, 220') sich über die Ebene der Sperrschicht (108; 206) hinaus erstreckt.

## Revendications

1. Article absorbant (10 ; 100 ; 200) comprenant :
un absorbant (11 ; 102 ; 208) ayant une surface côté corporel (12 ; 104) ;
un écran (14 ; 108 ; 206) imperméable aux liquides ; et
des bords de côté longitudinal (16,16' ; 110,112 ; 214,216)
caractérisé en ce que
lesdits bords de côté longitudinal (16,16' ; 110,112 ; 214,216) sont enroulés en spirale vers l'intérieur pour former des barrières de côté longitudinal (18,18' ; 114,116 ; 220,220') d'un seul tenant.

2. Article absorbant selon la revendication 1, dans lequel ledit écran imperméable aux liquides (14) forme une surface de l'article absorbant et dans lequel lesdits bords de côté longitudinal (16,16') s'étendent latéralement vers l'extérieur à partir d'un axe central longitudinal de la structure absorbante.

3. Article absorbant selon l'une des revendications précédentes, dans lequel ledit absorbant (102 ; 208) a une surface côté vêtement (106), ledit écran (108 ; 206) imperméable aux liquides est fixé à ladite surface côté vêtement (106), l'un desdits absorbant et écran ayant une largeur supérieure et s'étendant latéralement vers l'extérieur pour définir lesdits bords de côté longitudinal (110,112 ; 214,216).

4. Article absorbant selon l'une des revendications précédentes, dans lequel ledit absorbant (102) est plus large que ledit écran (108).

5. Article absorbant selon l'une des revendications 1 à 3, dans lequel ledit écran (108) est plus large que ledit absorbant (102).

6. Article absorbant selon l'une des revendications précédentes, dans lequel lesdits bords de côté longitudinal (110,112) sont enroulés en spirale vers l'intérieur en direction dudit absorbant (102) pour former lesdites barrières (114,116) d'un seul tenant.

7. Article absorbant selon l'une des revendications précédentes, comprenant en outre une enveloppe perméable aux liquides (126) fixée à ladite surface côté corporel (104) dudit absorbant (102).

8. Article absorbant selon l'une des revendications précédentes, dans lequel lesdites barrières (114,116) d'un seul tenant ont une portion supérieure extrême s'étendant au-dessus du plan dudit écran (108).

9. Article absorbant selon l'une des revendications précédentes, comprenant en outre des moyens de traction (218) pour conférer une courbure concave le long d'un axe longitudinal en direction de ladite surface côté corporel (204), lesdits moyens de traction (218) étant fixés à l'une au moins desdites barrières (220,220') d'un seul tenant.

10. Article absorbant selon l'une des revendications précédentes, comprenant en outre un élément résilient disposé entre ladite surface côté corporel (204) et ledit écran (206), dans lequel l'un au moins dudit premier élément, dudit écran (206) et de ladite couche résiliente (212) a une largeur supérieure et s'étend latéralement vers l'extérieur pour définir lesdits bords de côté longitudinal (214,216), lesdits bords étant enroulés en spirale vers l'intérieur pour former lesdites barrières de côté longitudinal (220,220') d'un seul tenant.

11. Article absorbant selon la revendication 1, comprenant en outre une enveloppe perméable aux liquides (126 ; 202) ayant une surface côté corporel (204), dans lequel ledit écran imperméable aux liquides a une surface côté vêtement (106), et dans lequel ledit absorbant (102 ; 208) est disposé entre ladite enveloppe (126 ; 202) et ledit écran (108 ; 206), ledit absorbant ayant des portions de côté longitudinal (107 ; 210), l'une au moins de ladite enveloppe (126 ; 202) et dudit écran (108 ; 206) s'étendant latéralement au-delà desdites portions de côté longitudinal (107 ; 210) pour former lesdits bords de côté longitudinal (110,112 ; 214,216).

12. Article absorbant selon l'une des revendications précédentes, dans lequel lesdits bords de côté longitudinal (110,112) sont enroulés en spirale vers l'intérieur en direction dudit écran (108).

13. Article absorbant selon l'une des revendications précédentes, dans lequel lesdits bords de côté longitudinal (110,112) sont enroulés en spirale en direction de ladite surface côté corporel (104) pour former ladite barrière.

14. Article absorbant selon l'une des revendications 7 à 13, dans lequel tant ladite enveloppe (126) que ledit écran (108) s'étendent latéralement au-delà desdites portions de côté longitudinal (107) pour former lesdits bords de côté longitudinal (110,112).

15. Article absorbant selon l'une des revendications 7 à 14, dans lequel lesdites barrières d'un seul tenant (114, 116) ont une portion supérieure extrême qui s'étend au-dessus du plan de ladite surface côté corporel de ladite enveloppe (126) .

16. Article absorbant selon l'une des revendications 7 à 15, comprenant en outre une couche résiliente (212) disposée entre ladite enveloppe (202) et ledit écran (206).

17. Article absorbant selon la revendication 16 dans lequel ladite couche résiliente (212) est hydrophile.

18. Article absorbant selon l'une des revendications 16 ou 17, dans lequel ladite couche résiliente (212) s'étend latéralement vers l'extérieur au-delà desdites portions de côté longitudinal (210) dudit absorbant (208) .

19. Article absorbant selon l'une des revendications 11 à 18, dans lequel ladite barrière de côté (114) d'un seul tenant comporte une ligne centrale verticale la divisant en un segment intérieur (122) et un segment extérieur (124), ledit segment intérieur (122) étant positionné à proximité de ladite portion de côté longitudinal (107) et ledit segment extérieur (124) étant positionné à l'écart de ladite portion de côté longitudinal (107), ledit segment intérieur (122) étant au moins partiellement hydrophile.

20. Article absorbant selon la revendication 19, dans lequel ledit segment intérieur (122) est positionné adjacent à ladite portion de côté longitudinal (107).

21. Article absorbant selon la revendication 19 ou 20, dans lequel ledit segment intérieur (122) est espacé d'une distance prédéterminée de ladite portion de côté longitudinal (107).

22. Article absorbant selon l'une des revendications 11 à 21, dans lequel lesdites portions de côté longitudinal (107) sont non linéaires.

23. Article absorbant selon l'une des revendications précédentes, comprenant en outre un moyen de traction (218) pour conférer une courbure concave le long d'un axe longitudinal en direction de ladite surface côté corporel (204), ledit moyen de traction (218) étant fixé à l'une au moins desdites barrières d'un seul tenant (220,220').

24. Article absorbant selon l'une des revendications précédentes, qui est une serviette hygiénique.

25. Article absorbant selon l'une des revendications 16 à 24, dans lequel ledit absorbant (208) est positionné adjacent à ladite couche résiliente (212), ledit absorbant ayant une portion de côté longitudinal (210) et l'une au moins de ladite enveloppe (202), dudit écran (206) et de ladite couche résiliente (212) s'étendant latéralement au-delà de ladite portion de côté longitudinal (210) pour former le bord de côté longitudinal (216), ledit bord de côté longitudinal (216) étant enroulé en spirale vers l'intérieur pour former une barrière de côté longitudinal (220) d'un seul tenant.

26. Article absorbant selon l'une des revendications précédentes, dans lequel ledit bord de côté longitudinal (216) est non linéaire.

27. Article absorbant selon l'une des revendications 25 ou 26, dans lequel lesdits côtés s'étendant latéralement sont enroulés en spirale vers l'intérieur en direction de ladite surface côté corporel (104 ; 204) et ladite barrière de côté (114,115 ; 220,220') d'un seul tenant s'étend au-dessus du plan dudit écran (108 ; 206).
